# EUROPEAN PATENT SPECIFICATION

(11) **EP 0 724 404 B1**
(45) Date of publication and mention of the grant of the patent: **27.05.1998**
(21) Application number: 94924818.1
(22) Date of filing: 29.07.1994
(51) Int. Cl.: A61B 6/14, G03B 17/48

(54) **APPARATUS FOR ACQUIRING IMAGES, PARTICULARLY FOR DENTAL RADIOLOGY**
BILDAUFNAHMEVORRICHTUNG, INSBESONDERE FÜR ZAHNÄRZTLICHE RÖNTGENAUFNAHMEN
APPAREIL D'ACQUISITION D'IMAGES, ET PLUS PARTICULIEREMENT, APPAREIL DE RADIOLOGIE DENTAIRE

(30) Priority: 17.09.1993 IT VE930026
(43) Date of publication of application: 07.08.1996
(73) Proprietor: O.M.S. - S.p.A. Officine Meccaniche Specializzate, 35020 Caselle di Selvazzano (IT)
(72) Inventor: SORDINA, Giuseppe, I-35100 Padova (IT); VILMART, Roland, I-35100 Padova (IT)
(74) Representative: Piovesana, Paolo
(86) International application number: EP9402512
(87) International publication number: WO9507656

(56) References cited:
- EP-A- 0 149 502
- DE-A- 3 622 843
- FR-A- 2 701 831
- GB-A- 681 158
- US-A- 3 051 166
- US-A- 4 587 555
- DATABASE WPI Week 9150, Derwent Publications Ltd., London, GB; AN 91-367473 & SU,A,1 621 873 (MOSC ARCHITECTURE) 23 January 1991

## Description

This invention relates to an apparatus for acquiring images, particularly for dental radiology.

Apparatus used in dental radiology are known for acquiring images which are then processed, displayed and possibly memorized for subsequent use.

A known apparatus is described in EP-B1-0129451. It comprises an intraoral container with a radiotransparent wall, on the inner surface of which there is applied a scintillator consisting of a layer of rare earths which transform the radiological image, formed when an X-ray beam strikes a structure to be radiographed interposed between an X-ray source and said container, into an optical image which is then fed via a converging bundle of optical fibres onto a CCD sensor facing said scintillator. The purpose of this CCD sensor, which is controlled by an electronic circuit also housed in the container, is to convert the optical image into an electronic signal which can be transferred to the outside of the container for its subsequent use.

A drawback of this arrangement is that the CCD sensor and its electronic control circuit, housed in the intraoral container, are sensitive to the X-rays passing through the radiotransparent wall of said container, because of which suitable shielding means have to be provided making the construction of the container considerably complicated and costly.

A further drawback of this known arrangement is that the CCD sensor and its electronic control circuit in practice constitute a telecamera constrained to this exclusive use, with evident limitation in its applications.

EP-A1-0364863 describes another known apparatus of this type. It comprises a container similar to the proceding, a scintillator, a CCD sensor and an electronic control circuit for the CCD sensor.

This arrangement differs from the preceding essentially by the different method of converging onto the CCD sensor the optical image which forms on the scintillator. In this respect, this arrangement uses a mirror facing the scintillator and arranged to reflect the optical image onto a lens system which causes the image to converge onto a CCD sensor. Hence again in this case shielding means for the CCD sensor and the relative electronic control circuit must be provided within the container and in addition both the CCD sensor and its electronic control circuit are constrained to this specific use.
EP-A-0149502 discloses an apparatus for acquiring images comprising the features of the preamble of claim 1, in particular a container having a radiotransparent wall with a scintillator, a tubular conduit extending from the container arranged at an angle to the wall, means for receiving the radiation formed within the scintillator, an optical system and a telecamera.

According to the invention an apparatus is provided for acquiring images, particularly for dental radiology, as claimed in claim 1.

A preferred embodiment of the present invention and a modification thereof are described in greater detail hereinafter by way of non-limiting example with reference to the accompanying drawings, in which:

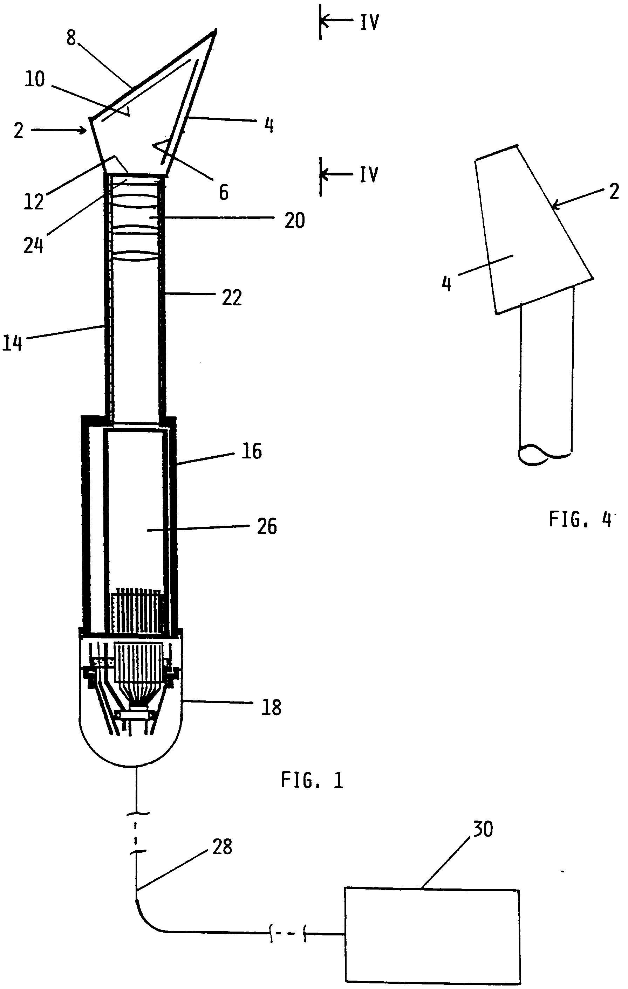

- Figure 1: is a partly sectional schematic side view of the apparatus according to the invention; and
- Figure 2: is a partial view of a modification thereof in the direction II-II of Figure 1.

As can be seen from the figures, the apparatus according to the invention comprises a container 2 of dimensions suitable for its comfortable introduction into the oral cavity of a patient. A wall 4 of this container 2 consists of a radiotransparent screen internally coated with a rare earth layer 6.

That wall 8 of the container 2 facing the wall 4 is inclined to this latter by about 40°, and carries a mirror 10 applied to its inner face. That wall 12 adjacent to the wall 4 and opposite the wall 8 comprises a circular aperture, to which there is applied a tubular conduit portion 14, the axis of which is inclined by about 10° to the normal to the wall 12. The tubular conduit portion 14 extends into a coaxial tubular conduit portion 16 of greater diameter, closed at its end by an axially withdrawable base 18. Into the tubular conduit portion 14 there is inserted an optical system 20 comprising a plurality of lenses contained in a single sleeve 22, which can be inserted axially into the conduit portion until it reaches a predetermined position defined by an inner circumferential rib 24.

A telecamera 26 fixed to the base 18 is inserted into the tubular conduit portion 16 into a position coaxially facing the optical system 20, and has its exit cable 28 passing through the base to be connected to an external computer 30.

The other side walls of the container 2 adjacent to the wall 4 form therewith an acute angle to facilitate the introduction of the container into the oral cavity of a patient.

Since the system for acquiring radiological images used in the apparatus according to the invention foresees a mutual positioning of the optical system 20 with respect to the light-sensitive element 32 (CCD) of the telecamera 26 extremely accurate (±0,01 mm for linear positionings and ±4′ for angular positionings) and indeed higher than that allowed from tolerances with which the components are provided by the respective manufacturers, the invention also foresees a system for the fine adjustment of such a mutual positioning.

So the CCD 32 of the telecamera 26 is mounted on an annular support 34 housed at the inside of the tubular conduit 36 of the telecamera 26, which conduit is in its turn slidably housed at the inside of the sleeve 16, which for this purpose is provided with a reference circumferential flange 38.

The connection between the annular support 34 of CCD 32 and the tubular conduit 36 of the telecamera 26 is obtained through three eccentrics 40, angularly spaced apart at 120° (see fig. 4) which enable to place, as it will be better clarified, the surface of the CCD exactly orthogonal to the optical axis of the optical system 20.

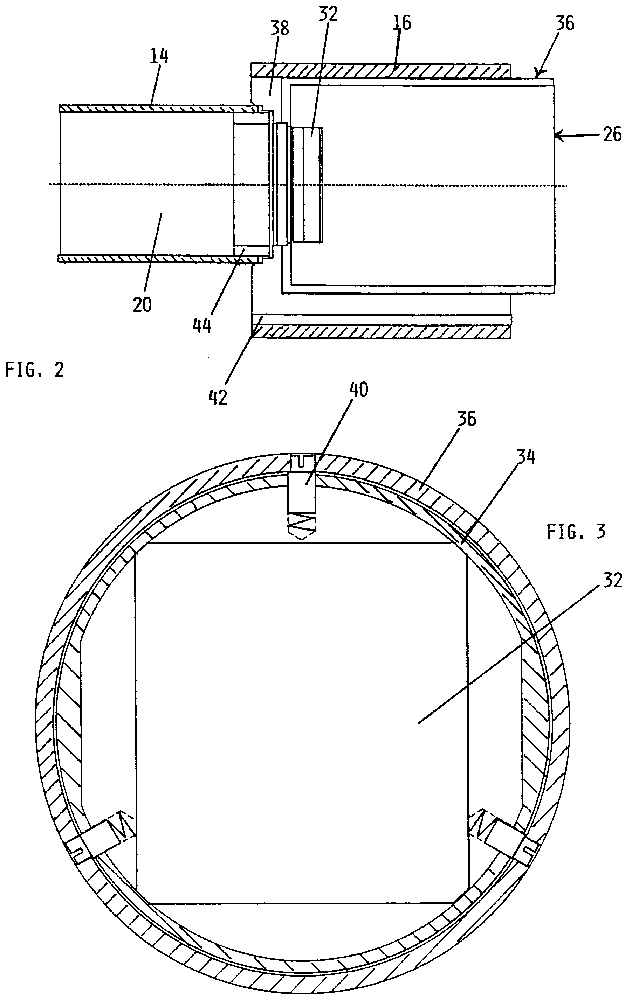

It has to be pointed out that the position of the conduit 36 of telecamera 24 with respect to the tubular conduit 14 is not concentric, but indeed it is off-line (see fig. 3) and this for the purpose of obtaining a longitudinal portion of said sleeve 16 suitable for the passage of a cable (not shown) and of a duct 42 to be used in the case the telecamera 26 is not used connected with the optical system 20 for the acquisition of radiological images, but is used, as it will be better clarified, with a different termination for endovision provided with a light source and requiring a jet of air for the cooling thereof.

The correct axial positioning of the optical system 20 with respect to the telecamera 26, so that the optical image provided by said optical system exactly forms on the surface of the CCD 32, is obtained through the interposition of a reference ring 44 between said optical system 20 and said sleeve 16, outwardly to the reference flange 38.

Practically the assembly of the apparatus according to the invention, foresees the following operations:
- firstly the optical system 20 is coupled to the container 2 in the correct mutual position, so that the normal to the wall 4, reflected from the mirror 10, is parallel to the optical axis of the optical system. Once this mutual position has been reached, the two parts are glued together,
- subsequently, after having determined through a suitable instrument system the distance between the optical system 20 and the telecamera 26 so as to have the formation of the image provided by the optical system exactly on the CCD 32, the reference ring 44 is prepared which determines with the required accuracy that distance, and then it is glued to the sleeve 22 housing the optical system,
- separately the angular position of CCD 32 is adjusted by keeping the telecamera inserted onto the sleeve 16 or onto other similar structure having a similar reference flange 38. This adjustment is obtained by acting on the three eccentrics 40 until to put the surface of CCD perfectly parallel to the reference plane of the flange 38,
- the telecamera 26, so adjusted, is introduced into the sleeve 16 until it rests to the reference flange 38 and then is blocked in such a position by screwing the threaded base 18.

The apparatus of the invention operates in the following manner:
after the container 2 has been introduced into the oral cavity of the patient such that the radiotransparent wall 4 rests against the arch region to be radiographed, the radiological apparatus is positioned on the opposite side of the arch to feed an X-ray beam through the arch so that it strikes the rare earth layer 6 to form a luminescent image therein.

This image is collected by the mirror 10 and reflected thereby onto the optical system 20, which focuses it onto the telecamera 26 where said image is converted into an electrical signal. From here this signal passes through the cable 28 to the computer 30 which reforms the image, displays it and possibly memorizes it. If the arch region to be radiographed is the distal region, the radiotransparent wall 4 of the container 2 can be applied to it practically without any difficulty by virtue of the inclination of this wall to the conduit, which remains outside the oral cavity.

To further facilitate the positioning of the container 2, this can not only be inclined to the axis of the conduit portions 14 and 16 in the plane perpendicular to the plane of the wall 4 but its centre line can also be inclined within this latter plane (see Figure 2).

If endoscopy of the oral cavity is required rather than radiography of an arch, it is possible to merely withdraw from the base 18 the assembly comprising the conduit portion 16 forming the enclosure for the telecamera 26, the conduit portion 14 with the optical system 20 housed within it and the container 2, and replace it with an endovisor, for example of fibre optic type, which in this manner is coupled to the telecamera 26. The so obtained device, however, does not form part of the present invention.

If instead an optical image for example of part of the oral cavity is required, after withdrawing the tubular conduit portion 16 from the base 18 as in the preceding case, a lens is coupled to the telecamera enabling images of the oral cavity or its exterior to be taken, which images can be transferred to the computer 30 for their processing, display and memorization.

It is apparent that the apparatus according to the invention is substantially more advantageous than traditional apparatus, in that:
- having transferred the CCD sensor and its electronic control circuit to outside the container 2, any risk of their damage by X-rays is eliminated, even without any shielding,
- constructional costs are substantially reduced, both because of having dispensed with the need for shielding the CCD sensor and its electronic control circuit as stated, and because of the more simple construction of the intraoral container which, because of its size, is difficult to assemble,
- any region of the oral cavity can be reached with ease and comfort, both for the dentist and for the patient, and
- the telecamera can also be used for other applications for which the traditional art requires separate apparatus, each provided with its own telecamera.

## Claims

1. An apparatus for acquiring images, particularly for dental use, comprising:
- a telecamera (26) to be connected to an external computer (30), housed in a first tubular conduit (16) provided with a base (18) at its proximal end, and
- a second tubular conduit (14), extending into the first tubular conduit (16) and optically facing said telecamera (26),
- the second tubular conduit (14) being in contact with a container (2) of dimensions suitable for its introduction into the oral cavity of a patient, comprising a radiotransparent wall (4), to the inner surface of which there is applied a scintillator (6) converting the incident X-rays into visible radiation, wherein a container wall (12) in contact with the second tubular conduit (14) is arranged at an angle to said radiotransparent wall (4), the container (2) further comprising means (10) for receiving the visible radiation formed within said scintillator (6) and for conveying it into said second tubular conduit (14) parallel to its axis through an optical system (20) facing the telecamera (26) housed within a portion of the said second tubular conduit (14) which is close to said container (2), characterised in that the base (18) is withdrawable and threaded so that the telecamera (26) is blocked in its position in the first tubular conduit (16) by screwing the threaded base (18) thereon.

2. An apparatus as claimed in claim 1, characterised by comprising within the container (2) a mirror (10) arranged to reflect onto said optical system (20) the optical image formed on said scintillator (6).

3. An apparatus as claimed in claim 1, characterised by comprising a converging bundle of optical fibres, of which the input termination is housed within said container (2) in a position facing said scintillator (6) and the exit termination is housed within said second tubular conduit (14) in a position facing said optical system (20).

4. An apparatus as claimed in claim 1, characterised in that the axis of said second tubular conduit (14) lies at an angle to the surface of the radiotransparent wall (4).

5. An apparatus as claimed in claim 1, characterised in that the axis of said second tubular conduit (14) forms an angle of between 7° and 15°, and preferably 10°, with the surface of the radiotransparent wall (4).

6. An apparatus as claimed in claim 1, characterised in that the longitudinal centre line of the radiotransparent wall (4) lies at an angle to the axis of said second tubular conduit (14).

7. An apparatus as claimed in claim 6, characterised in that the angle formed between the longitudinal centre line of the radiotransparent wall (4) and the axis of said second tubular conduit (14) is between 7° and 15°, and preferably 10°.

8. An apparatus as claimed in claim 1, characterised in that at least part of the container walls form an acute angle with the radiotransparent wall (4).

9. An apparatus as claimed in claim 1, characterised in that said second tubular conduit (14) housing the optical system (20) has a diameter less than the diameter of said first tubular conduit (16) housing the telecamera (26).

10. An apparatus as claimed in claim 1 characterised in that said first tubular conduit (16) housing the telecamera (26) is provided with a reference flange (38) to which the telecamera (26) rests to define the correct position with respect to the optical system (20).

11. An apparatus as claimed in claim 10 characterised in that the sensor CCD (32) of the telecamera is mounted on an angular support (34) bound to the tubular conduit (36) of the telecamera (26) through elements (40) for the micrometric adjustment.

12. An apparatus as claimed in claim 11 characterised in that the annular support (34) is bounded to the tubular conduit (36) of the telecamera (26) through eccentrics angularly spaced apart along the circumference of said conduit.

13. An apparatus as claimed in claim 10 characterised in that it comprises a reference ring (44) placed between the optical system (20) and the reference flange (38) of said first tubular conduit (16) to determine the correct distance between said optical system and the sensor CCD (32) of the telecamera (26) resting against the opposite side of said reference flange (38).

## Patentansprüche

1. Bildaufnahmevorrichtung, insbesondere zur zahnärztlichen Verwendung, umfassend:
- eine Fernsehkamera (26) zum Anschluss an einen externen Computer (30), welche in einer ersten rohrförmigen Leitung (16) untergebracht ist, welche mit einer Basis (18) an ihrem benachbarten Ende versehen ist, und
- eine zweite rohrförmige Leitung (14), die sich in die erste rohrförmige Leitung (16) erstreckt und der besagten Kamera (26) optisch gegenüberliegt, wobei
- die zweite rohrförmige Leitung (14) mit einem Behälter (2) mit zur Einführung in die Mundhöhle eines Patienten geeignete Abmessungen in Kontakt steht, welcher eine strahlungstransparente Wand (4) umfaßt, an deren innerer Oberfläche ein Szintillator (6) aufgebracht ist, der die einfallenden Röntgenstrahlen in sichtbare Strahlung umwandelt, wobei eine Behälterwand (12) in Kontakt mit der zweiten rohrförmigen Leitung (14) in einem Winkel zu der besagten strahlungstransparenten Wand (4) angeordnet ist, wobei der Behälter (2) außerdem Mittel (10) zum Empfang der sichtbaren Strahlung, die in dem besagten Szintillator (6) gebildet wird und zu deren Weiterleitung in die besagte zweite rohrförmige Leitung (14) parallel zu deren Achse durch ein optisches System (20) aufweist, das der Fernsehkamera (26), die in einem Abschnitt der besagten zweiten rohrförmigen Leitung (14) untergebracht ist, welcher dem besagten Container (2) benachbart ist, gegenüberliegt, dadurch gekennzeichnet, dass die Basis (18) abziehbar und mit einem Gewinde versehen ist, so dass die Fernsehkamera (26) in ihrer Position in der ersten rohrförmigen Leitung (16) blockiert wird, indem die mit einem Gewinde versehene Basis (18) auf sie geschraubt wird.

2. Vorrichtung nach Anspruch 1, dadurch gekennzeichnet, dass sie in dem Behälter (2) einen Spiegel (10) aufweist, der so angeordnet ist, dass auf das besagte optische System (20) das optische Bild reflektiert wird, das auf dem besagten Szintillator (6) gebildet wird.

3. Vorrichtung nach Anspruch 1, dadurch gekennzeichnet, dass sie ein konvergierendes Bündel von optischen Fasern aufweist, von dem das Einlaßende in dem besagten Container (2) in einer Position untergebracht ist, die dem besagten Szintillator (6) gegenüberliegt, und das Auslaßende in der besagten zweiten rohrförmigen Leitung (14) in einer Position untergebracht ist, die dem besagten optischen System (20) gegenüberliegt.

4. Vorrichtung nach Anspruch 1, dadurch gekennzeichnet, dass die Achse der besagten zweiten rohrförmigen Leitung (14) in einem Winkel zur Oberfläche der strahlungstransparenten Wand (4) liegt.

5. Vorrichtung nach Anspruch 1, dadurch gekennzeichnet, dass die Achse der besagten zweiten rohrförmigen Leitung (14) einen Winkel zwischen 7° und 15°, vorzugsweise von 10°, mit der Oberfläche der strahlungstransparenten Wand (4) bildet.

6. Vorrichtung nach Anspruch 1, dadurch gekennzeichnet, dass die Längsmittellinie der strahlungstransparenten Wand (4) in einem Winkel zur Achse der besagten zweiten rohrförmigen Leitung (14) liegt.

7. Vorrichtung nach Anspruch 6, dadurch gekennzeichnet, dass der Winkel, der zwischen der Längsmittellinie der strahlungstransparenten Wand (4) und der Achse der besagten zweiten rohrförmigen Leitung (14) gebildet wird, zwischen 7° und 15°, vorzugsweise 10°, beträgt.

8. Vorrichtung nach Anspruch 1, dadurch gekennzeichnet, dass wenigstens ein Teil der Behälterwände einen spitzen Winkel mit der strahlungstransparenten Wand (4) bildet.

9. Vorrichtung nach Anspruch 1, dadurch gekennzeichnet, dass die besagte zweite rohrförmige Leitung (14), die das optische System (20) aufnimmt, einen Durchmesser aufweist, der kleiner ist als der Durchmesser der besagten ersten rohrförmigen Leitung (16), welche die Fernsehkamera (26) aufnimmt.

10. Vorrichtung nach Anspruch 1, dadurch gekennzeichnet, dass die besagte erste rohrförmige Leitung (16), die die Fernsehkamera (26) aufnimmt, mit einem Referenzflansch (38) versehen ist, an dem die Fernsehkamera (26) anliegt, um die korrekte Position in Bezug auf das optische System (20) zu definieren.

11. Vorrichtung nach Anspruch 10, dadurch gekennzeichnet, dass der Sensor CCD (32) der Fernsehkamera, auf einen Winkelträger (24) montiert ist, der mit der rohrförmigen Leitung (36) der Fernsehkamera (26) durch Elemente (40) zur Mikrometer-Einstellung verbunden ist.

12. Vorrichtung nach Anspruch 11, dadurch gekennzeichnet, dass der Winkelträger (34) mit der rohrförmigen Leitung (36) der Fernsehkamera (26) durch Exzenter verbunden ist, die im Winkelabstand um den Umfang der besagten Leitung angeordnet sind.

13. Vorrichtung nach Anspruch 10, dadurch gekennzeichnet, dass sie einen Referenzring (44) aufweist, der zwischen dem optischen System (20) und dem Referenzflansch (38) der besagten ersten rohrförmigen Leitung (16) angeordnet ist, um den korrekten Abstand zwischen dem besagten optischen System und dem Sensor CCD (32) der Fernsehkamera (26) zu bestimmen, welche an der gegenüberliegenden Seite des besagten Referenzflansches (38) anliegt.

## Revendications

1. Un appareil d'acquisition d'images, et plus particulièrement à usage dentaire, comprenant :
- une télécaméra (26) à relier à un ordinateur extérieur (30), logé dans un premier conduit tubulaire (16) muni d'une embase (18) à son extrémité proximale, et
- un deuxième conduit tubulaire (14), s'étendant dans le premier conduit tubulaire (16) et faisant face optiquement à ladite télécaméra (26),
- le deuxième conduit tubulaire (14) étant en contact avec une ampoule (2) de dimensions convenables pour son introduction dans la cavité buccale d'un patient, comprenant une paroi radiotransparente (4) sur la surface interne de laquelle il est appliqué un scintillateur (6) convertissant les rayons X incidents en radiation visible, une paroi d'ampoule (12) en contact avec le deuxième conduit tubulaire (14) étant disposée avec un angle par rapport à ladite paroi radiotransparente (4), l'ampoule (2) comprenant en outre un moyen (10) pour recevoir la radiation visible formée à l'intérieur dudit scintillateur (6) et pour la transmettre dans ledit deuxième conduit tubulaire (14) parallèlement à son axe à travers un système optique (20) faisant face à la télécaméra (26) logée dans une portion dudit deuxième conduit tubulaire (14) qui est adjacent à ladite ampoule (2), caractérisé en ce que l'embase (18) est retirable et filetée afin que la télécaméra (26) soit bloquée à sa position dans le premier conduit tubulaire (16) par vissage de l'embase filetée (18) sur celui-ci.

2. Un appareil comme revendiqué dans la revendication 1, caractérisé en ce qu'il comprend dans l'ampoule (2) un miroir (10) agencé pour réfléchir vers ledit système optique (20) l'image optique formée sur ledit scintillateur (6).

3. Un appareil comme revendiqué dans la revendication 1, caractérisé en ce qu'il comprend un faisceau convergent de fibres optiques, dont la terminaison d'entrée est logée à l'intérieur de ladite ampoule (2) à une position faisant face audit scintillateur (6) et la terminaison de sortie est logée à l'intérieur dudit deuxième conduit tubulaire (14) à une position faisant face audit système optique (20).

4. Un appareil comme revendiqué dans la revendication 1, caractérisé en ce que l'axe dudit deuxième conduit tubulaire (14) fait un angle avec la surface de la paroi radiotransparente (4).

5. Un appareil comme revendiqué dans la revendication 1, caractérisé en ce que l'axe dudit deuxième conduit tubulaire (14) forme un angle entre 7° et 15°, et de préférence 10°, avec la surface de la paroi radiotransparente (4).

6. Un appareil comme revendiqué dans la revendication 1, caractérisé en ce que la ligne centrale longitudinale de la paroi radiotransparente (4) fait un angle avec l'axe dudit deuxième conduit tubulaire (14).

7. Un appareil comme revendiqué dans la revendication 6, caractérisé en ce que l'angle formé entre la ligne centrale longitudinale de la paroi radiotransparente (4) et l'axe dudit deuxième conduit tubulaire (14) est entre 7° et 15°, et de préférence 10°.

8. Un appareil comme revendiqué dans la revendication 1, caractérisé en ce qu'au moins une partie des parois d'ampoule forme un angle aigu avec la paroi radiotransparente (4).

9. Un appareil comme revendiqué dans la revendication 1, caractérisé en ce que ledit deuxième conduit tubulaire (14) logeant le système optique (20) a un diamètre inférieur au diamètre dudit premier conduit tubulaire (6) logeant la télécaméra (26).

10. Un appareil comme revendiqué dans la revendication 1, caractérisé en ce que ledit premier conduit tubulaire (16) logeant la télécaméra (26) est prévu avec une bride de référence (38) sur laquelle la télécaméra (26) repose pour définir la position correcte par rapport au système optique (20).

11. Un appareil comme revendiqué dans la revendication 10, caractérisé en ce que le capteur CCD (32) de la télécaméra est monté sur un support angulaire (34) lié au conduit tubulaire (36) de la télécaméra (26) par des éléments (40) pour réglage micrométrique.

12. Un appareil comme revendiqué dans la revendication 11, caractérisé en ce que le support annulaire (34) est lié au conduit tubulaire (36) de la télécaméra (26) par des excentriques répartis angulairement le long de la périphérie dudit conduit.

13. Un appareil comme revendiqué dans la revendication 10, caractérisé en ce qu'il comprend une bague de référence (44) placée entre le système optique (20) et la bride de référence (38) dudit premier conduit tubulaire (16) pour déterminer la distance correcte entre ledit système optique et le capteur CCD (32) de la télécaméra (26) reposant contre la face opposée de ladite bride de référence (38).
